# EUROPÄISCHE PATENTANMELDUNG

(11) **EP 3 357 905 A1**
(43) Veröffentlichungstag der Anmeldung: **08.08.2018**
(21) Anmeldenummer: 17020041.4
(22) Anmeldetag: 01.02.2017
(51) Int. Cl.: C07C 269/00, C07C 271/28, C07D 231/22

(54) **HERSTELLUNG N-SUBSTITUIERTER AROMATISCHER HYDROXYLAMINE**

(71) Anmelder: Solvias AG, 4303 Kaiseraugst (CH)
(72) Erfinder: Steiner, Heinz, 4416 Bubendorf (CH)
(74) Vertreter: Althaus, Roland

(57) **Zusammenfassung**

Wirtschaftliches, einstufiges Verfahren zur Herstellung N-substituierter aromatischer Hydroxylamine der Formel I

R-N(OH)-C(=O)-(O)R₁ (I),

mit Wasserstoff durch katalytische Hydrierung mit gegebenenfalls modifizierten Hydrierkatalysatoren in einem aprotischen Lösungsmittel und in Gegenwart eines Halogenameisensäureesters und gegebenenfalls in Gegenwart einer Base.

## Beschreibung

Die vorliegende Erfindung betrifft ein Verfahren zur Herstellung N-Alkoxycarbonyl- oder N-Aryloxycarbonyl-substituierter aromatischer Hydroxylamine durch katalytische Hydrierung mit Wasserstoffgas und mit gegebenenfalls modifizierten Hydrierkatalysatoren in einem inerten Lösungsmittel und in Gegenwart eines Halogenameisensäureesters und gegebenenfalls einer Base.

N-substituierte aromatische Hydroxylamine sind wichtige Chemikalien, zum Beispiel zur Herstellung von Pestiziden. Die Herstellung erfolgt mittels 2-Stufen Reaktion, d.h. dass zuerst das aromatische Hydroxylamin hergestellt und dieses in einem nachfolgenden Schritt am Stickstoff substituiert wird. Die Herstellung der Arylhydroxylamine erfolgt häufig mittels katalytischer Hydrierung, katalytischer Transferhydrierung (zum Beispiel mit einem Rhodium Katalysator und Hydrazin als Wasserstoffdonor) oder Zink-Reduktion von Nitroaromaten. Hierbei bilden sich oft Nebenprodukte wie aromatische Amine sowie Azoxy-, Azo- und Hydrazoverbindungen. Es gab daher Bemühungen, diese Nebenreaktionen zu minimieren und damit die Ausbeute zu verbessern.

A. Porzelle et al. beschreibt in Synlett 2009, Nr. 5, Seiten 798 bis 802 die Herstellung N-substituierter Hydroxylaminen mittels einer Redoxreaktion. Nitroaromaten werden mit Zink und Ammoniumchlorid reduziert, wobei das Reaktionsgemisch Chlorameisensäureester enthält, um mittels Carbamatbildung das aromatische Hydroxylamin abzufangen und eine Überreduktion zum Amin zu unterdrücken. Die Reaktion ist jedoch unspezifisch. Es werden unerwünschte Gemische unterschiedlicher Zusammensetzung aus N-mono und N,0-disubstituierten Hydroxylaminen gebildet. Dieses Verfahren eignet sich daher nicht zur Herstellung N-substituierter Arylhydroxylamine im industriellen Massstab.

In der WO 2016/181386 wird die Herstellung des Zwischenproduktes der Formel A 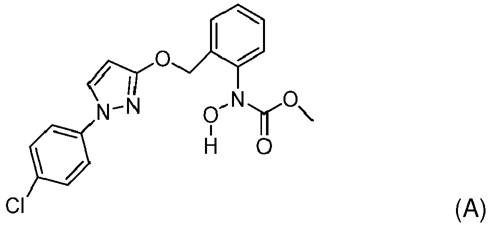 für das Fungizid Pyraclostrobin mittels eines Zweistufenverfahrens beschrieben. Die entsprechende Nitroverbindung wird zuerst mit einem Platinkatalysator in Gegenwart einer Stickstoffbase und einer Schwefelverbindung mit Wasserstoff hydriert und in einer zweiten Stufe mit Chlorameisensäuremethylester umgesetzt. Das Verfahren ist noch nicht ausreichend wirtschaftlich, da zwei nacheinander folgende chemische Stufen erforderlich sind und die Ausbeute noch nicht optimal ist.

Es besteht ein grosses Bedürfnis an einem ökonomischen, direkten, katalytischen Hydrierverfahren zur Herstellung von aromatischen N-substituierten Hydroxylaminen in einem einzigen Verfahrensschritt, mit dem hohe Selektivitäten und hohe Ausbeuten erzielt werden können. Ausserdem ist es sehr wünschenswert, die Akkumulation der häufig unstabilen und toxischen Arylhydroxylamine zu vermeiden. Aufgabe vorliegender Erfindung ist die Bereitstellung eines solchen Verfahrens.

Es wurde nun überraschend gefunden, dass die Anwesenheit von Halogenameisensäureestern während der katalytischen Hydrierung von aromatischen Nitroverbindungen toleriert wird, d.h. dass erstere praktisch nicht hydriert werden, und ein solches Verfahren in hohen bis sehr hohen Selektivitäten und Ausbeuten und zum gewünschten N-substituierten aromatischen Hydroxylamin führt. Nebenprodukte wie z.B. Azoxyverbindungen, N-substituierte aromatische Amine oder N,O-disubstituierte Arylhydroxylamine werden nur in geringem Ausmass gebildet.

Gegenstand vorliegender Erfindung ist ein Verfahren zur Herstellung von N-substituierten aromatischen Hydroxylaminen der Formel I

R-N(OH)-C(=O)-(O)R₁ (I),

worin R ein aromatischer Rest ist und R₁ einen Kohlenwasserstoffrest bedeutet, durch Hydrierung von aromatischen Nitroverbindungen der Formel II

R-NO₂ (II),

worin R die zuvor angegebene Bedeutung hat, mit einem Hydrierkatalystor und Wasserstoff in einem aprotischen Lösungsmittel, das dadurch gekennzeichnet ist, dass man die Reaktion in Anwesenheit wenigstens stöchiometrischer Mengen einer Verbindung der Formel III

Y-C(=O)-(O)R₁ (III),

worin Y Halogen ist und R₁ die zuvor angegebene Bedeutung hat, durchführt.

Bei der Gruppe R kann es sich um aromatische Kohlenwasserstoffe oder Heteroaromaten handeln, deren Ringsysteme kondensiert oder verknüpft und unsubstituiert oder substituiert sein können. Einige Beispiele für aromatische Kohlenwasserstoffe sind Benzole wie z.B. 2-Nitrotoluol, Naphthalin oder Anthracen, polycyclische Kohlenwasserstoffe (auch teilhydrierte wie Tetralin), Biphenyl, Cyclopentadienyl-Anion und Anthrachinon. Einige Beispiele für Heteroaromaten sind Pyridine, Pyrrole, Azole, Diazine, Triazine, Triazole, Furane, Thiophene, Oxazole, Indole, Chinoline, Isochinoline, Carbazole, Purine, Phtalazine, Benztriazole, Benzofurane, Chinazole, Acridine und Benzothiophene.

Die Gruppe R kann substituiert sein, auch mit Substituenten, die grundsätzlich ebenfalls hydriert werden können. Wenn eine solche Reaktion erwünscht ist, kann man entsprechende Katalysatoren verwenden. Falls es unerwünscht ist, können entweder *per se* selektive Katalysatoren oder modifizierte Katalysatoren eingesetzt werden.

Die Gruppe R kann gleiche oder verschiedene Substituenten wie Halogen, Hydroxyl, oder einen über ein C-Atom, O-Atom, S-Atom, N-Atom, P-Atom oder Si-Atom oder Gruppen CO, C(O)O, SO, SO₂, gebundenen Kohlenwasserstoffrest zum Beispiel ein- bis sechsmal, bevorzugt ein- bis viermal und besonders bevorzugt einbis zweimal enthalten, wobei Kohlenwasserstoffreste in den Substituenten ihrerseits substituiert sein können. Die cyclischen Reste R können auch mit ringbildenden Substituenten versehen sein, zum Beispiel C₂-C₄-Alkylen, C₂-C₄-Alkenylen, C₄-C₈-Alkdienylen, C₁-C₂-Alkylendiamino oder C₁-C₂-Alkylendioxyl.

Bei dem gegebenenfalls substituierten Substituenten kann es sich zum Beispiel um C₁-C₁₂-Alkyl, bevorzugt C₁-C₈-Alkyl und besonders bevorzugt C₁-C₄-Alkyl handeln. Beispiele sind Methyl, Ethyl, n- oder i-Propyl, n-, i- oder t-Butyl, Pentyl,
Hexyl, Octyl, Decyl, Undecyl und Dodecyl.

Bei dem gegebenenfalls substituierten Substituenten kann es sich zum Beispiel um C₃-C₈-Cycloalkyl, bevorzugt C₃-C₆-Cycloalkyl handeln. Beispiele sind Cyclopentyl, Cyclohexyl und Cyclopropyl.

Bei dem gegebenenfalls substituierten Substituenten kann es sich zum Beispiel um C₃-C₈-Cycloalkyl-alkyl, bevorzugt C₃-C₆-Cycloalkyl-alkyl mit zum Beispiel 1 bis 4 C-Atomen im Alkyl handeln. Beispiele sind Cyclopentylmethyl, Cyclohexylmethyl oder -ethyl und Cyclopropylmethyl.

Bei dem gegebenenfalls substituierten Substituenten kann es sich zum Beispiel um C₆-C₁₈-Aryl und bevorzugt C₆-C₁₀-Aryl handeln. Beispiele sind Phenyl oder Naphthyl.

Bei dem gegebenenfalls substituierten Substituenten kann es sich zum Beispiel um C₇-C₁₂-Aralkyl handeln, zum Beispiel Benzyl oder 1-Phenyleth-2-yl.

Bei dem gegebenenfalls substituierten Substituenten kann es sich zum Beispiel um Tri(C₁-C₄-Alkyl)Si oder Triphenylsilyl handeln. Beispiele für Trialkylsilyl sind Trimethyl-, Triethyl-, Tri-n-propyl-, Tri-n-Butyl- und Dimethyl-t-butylsilyl.

Bei dem Substituenten kann es sich zum Beispiel um Halogen handeln. Beispiele sind F und Cl.

Bei dem gegebenenfalls substituierten Substituenten kann es sich zum Beispiel um einen Alkyloxyrest, Dialkylaminorest oder Alkylthiorest handeln, worin Alkylgruppen C₁-C₁₂-Alkyl, bevorzugt C₁-C₈-Alkyl und besonders bevorzugt C₁-C₄-Alkyl bedeuten. Andere mögliche Substituenten sind C₅-C₈-Cycloalkyloxy oder - thio, bevorzugt C₅-C₆-Cycloalkyloxy oder -thio; (C₅-C₈-Cycloalkyl)₂N-, C₆-C₁₈-Aryloxy oder -thio und bevorzugt C₆-C₁₀-Aryloxy oder -thio; oder C₇-C₁₂-Aralkyloxy -thio bedeutet. Beispiele für die Kohlenwasserstoffreste in den Substituenten sind zuvor erwähnt worden.

Die Kohlenwasserstoffreste der Substituenten können ihrerseits ein- oder mehrfach, zum Beispiel ein- bis dreifach substituiert sein, zum Beispiel mit Halogen (F oder Cl, besonders F), -N(C₁-C₄-Alkyl)₂, C₁-C₄-Alkyl, C₁-C₄-Alkoxy, C₁-C₄-Alkylthio, C₅-C₆-Cycloalkyl, Phenyl, Benzyl, Phenoxy oder Benzyloxy.

R₁ kann als Kohlenwasserstoffrest bevorzugt Reste, ausgewählt aus der Gruppe lineares oder verzweigtes C₁-C₈-, bevorzugt C₁-C₆- und besonders bevorzugt C₁-C₄-Alkyl, C₄-C₈- und bevorzugt C₃-C₆-Cycloalkyl, zum Beispiel Cyclopentyl oder Cyclohexyl, Benzyl oder Phenyl bedeuten.

Besonders bevorzugt bedeutet R₁ C₁-C₄-Alkyl.

Bei den Verbindungen der Formel III handelt es sich um Halogenameisensäureester, wobei Halogen bevorzugt für Brom und insbesondere bevorzugt für Chlor steht.

In einer bevorzugten Ausführungsform handelt es sich bei den Verbindungen der Formel III um C₁-C₄-AlkylO-CO-Cl, ganz besonders bevorzugt um Chlorameisensäuremethylester.

Die Hydrierung kann in Gegenwart eines inerten aprotischen und apolaren oder polaren Lösungsmittels durchgeführt werden, wobei ein Lösungsmittel oder Gemische von Lösungsmitteln eingesetzt werden können. Geeignete Lösungsmittel sind zum Beispiel aliphatische, cycloaliphatische und aromatische Kohlenwasserstoffe (Pentan, Hexan, Petrolether, Cyclohexan, Methylcyclohexan, Benzol, Toluol, Xylol), aliphatische und aromatische Halogenkohlenwasserstoffe (Dichlormethan, Chloroform, Chlorbenzol), Nitrile (Acetonitril, Propionitril, Benzonitril), Ether (Diethylether, Dibutylether, Diisopropylether, *tert-*Butylmethylether, *tert*-Butylethylether, t-Amylmethylether, Cyclopentylmethylether, Ethylenglykoldimethylether, Ethylenglykoldiethylether, Diethylenglykoldimethylether, Tetrahydrofuran, 2-Methyltetrahydrofuran, Dioxan, Anisol), Kohlensäureester (Dimethylcarbonat), Carbonsäureester und Lactone (Essigsäuremethyl-, -ethyl- oder -isopropylester, Valerolacton), N-substituierte Lactame (N-Methylpyrrolidon), Carbonsäureamide (Dimethylacetamid, N-Methylpyrrolidon), acyclische Harnstoffe (N,N-Dimethyl-2-imidazolidinon), Sulfoxide (Dimethylsulfoxid) und Sulfone (Dimethylsulfon, Tetramethylensulfon). Dem Reaktionsgemisch kann auch Wasser zugesetzt werden zum Beispiel in einer Menge von bis zu 50 Gew.-% und bevorzugter bis zu 20 Gew.-%, bezogen auf die Menge des Lösungsmittels. Bevorzugte Lösungsmittel sind Ether, Kohlenwasserstoffe, halogenierte Kohlenwasserstoffe, Carbonsäureester, Carbonate und Amide. Besonders bevorzugt sind Ether, insbesondere Tetrahydrofuran, 2-Methyltetrahydrofuran, Cyclopentylmethylether, *tert-*Butylmethylether, *tert*-Butylethylether und *tert*-Amylmethylether.

Als Katalysatoren können (handelsübliche) Edelmetallkatalysatoren oder auch Basismetall-Katalysatoren verwendet werden. Geeignete Edelmetalle sind Ruthenium, Rhodium, Iridium, Palladium und Platin. Bevorzugt sind Palladium und insbesondere Platin. Die Edelmetalle sind üblicherweise auf Trägermaterialien aufgebracht und enthalten zum Beispiel bis zu 20 Gew.-% Edelmetall, bezogen auf das Trägermaterial. Trägermaterialien sind insbesondere Kohlenstoff, Metalloxide und Silikate. Geeignete Basismetall-Katalysatoren bestehen meist aus Nickel oder Kobalt, welche gegebenenfalls auf einem Trägermaterial wie z.B. Siliziumdioxid aufgebracht sind. In neuerer Zeit sind auch Methoden zur Herstellung von Katalysatoren in Form von Nanopartikeln bekannt geworden, sei es bei Edelmetall- oder bei Basismetallkatalysatoren. Auch solche Katalysatoren können im erfindungsgemässen Verfahren verwendet werden.

Die Katalysatoren werden in der Regel in Mengen von 0,1 bis 10 Gew.-%, bevorzugter 0,1 bis 5 Gew.-% verwendet, bezogen auf das Substrat.

Die meisten handelsüblichen Katalysatoren hydrieren die aromatische Nitrogruppe zur Aminogruppe, ohne dass das Arylhydroxylamin in hoher Selektivität gebildet wird. Es wurde nun gefunden, dass die Weiterhydrierung des intermediären Arylhydroxylamins unterdrückt werden kann, indem die katalytische Hydrierung in Gegenwart eines Halogenameisensäureesters durchgeführt wird. Die Gegenwart eines Halogenameisensäureesters sowie die in-situ Umwandlung der Hydroxylamino-Gruppe in eine N-Alkoxycarbonyl- oder N-Aryloxycarbonyl-Hydroxylaminogruppe führen offensichtlich zu einer stark verminderten Hydrogenolysierbarkeit der N-0 Bindung und somit zu einer stark erhöhten Selektivität. Überraschenderweise werden selbst bei Verwendung von Katalysatoren, welche normalerweise Nitroaromaten nicht in hoher Selektivität zu Arylhydroxylaminen hydrieren, hohe Selektivitäten an N-substituierten Arylhydroxylaminen erhalten.

Halogenameisensäureester haben eine ähnliche Reaktivität wie Carbonsäurehalogenide, welche durch Katalytische Hydrierung sehr leicht in Aldehyde umgewandelt werden können. Es ist daher überraschend und es war nicht vorhersehbar, dass Halogenameisensäureester während der erfindungsgemässen katalytischen Hydrierung aromatischer Nitroverbindungen nicht selbst in nennenswerter Weise hydriert werden. Dadurch ist es möglich, Halogenameisensäureester in stöchiometrischen bis leicht überstöchiometrischen Mengen zu verwenden.

Selbstverständlich können auch *per se* selektive oder modifizierte Katalysatoren, welche geeignet sind, die Hydrierung der Nitrogruppe auf der Stufe der Arylhydroxylamine anzuhalten, zu verwenden. Beispiele solcher Modifikatoren sind Schwefelverbindungen (zum Beispiel Sulfoxide, Thioether, Thiole, Thioharnstoff, Thiophen), Phosphorverbindungen (zum Beispiel Phosphine, Phosphinoxide, Phosphor- und Phosphonsäuren oder -ester), gegebenenfalls in Kombination mit Säuren (Essigsäure, Methansulfonsäure) oder tertiären Amine (N-Methylmorpholin, Tetramethylenethylendiamin). Bevorzugt sind Dimethylsulfoxid und insbesondere Hypophosphorige Säure.

Die Kombination eines möglichst selektiven, gegebenenfalls modifizierten Hydrierkatalysators mit der Verwendung eines Halogenameisensäureesters während der Hydrierung führt zu besonders hoher Selektivität in Bezug auf die Unterdrückung der Hydrogenolyse der N-0 Bindung und der Bildung von Azoxy- oder Hydrazoverbindungen.

Im erfindungsgemässen Verfahren wird hypophosphorige Säure H₃PO₂ oder Dimethylsulfoxid (DMSO) oder eine Kombination davon als Modifikator verwendet, weil mit deren Zusatz die gewünschten ArylN(OH)COOR in besonders hoher Selektivität und Ausbeute erhalten werden. Die Weiterhydrierung der Arylhydroxylamine und der ArylN(OH)COOR wird hiedurch besonders stark unterdrückt, ohne dass die Hydrieraktivität übermässig reduziert wird. Im Übrigen tolerieren H₃PO₂ und DMSO im Gegensatz zu Modifikatoren aus der Gruppe "Ammoniak, primäre und sekundäre Amine" die Verwendung eines Halogenameisensäureesters, weil erstere im Gegensatz zu den genannten Stickstoffverbindungen nicht mit diesem reagieren.

Der Einsatz von H₃PO₂ als Modifikator ist ausserdem besonders attraktiv, weil bei Verwendung von mit hypophosphoriger Säure modifizierter Edelmetall-Katalysatoren die katalytische Hydrierung anderer Funktionalitäten besonders stark gehemmt wird (siehe EP-B1-0 931 053). Somit können N-substituierte Arylhydroxylamine nicht nur praktisch frei von N-substituierter Arylaminen und von Azoxy- und Hydrazoverbindungen hergestellt werden, sondern auch unter grösstmöglicher Schonung weiterer hydrierbarer Funktionalitäten.

Eine bevorzugte Ausführungsform des erfindungsgemässen Verfahrens ist dadurch gekennzeichnet, dass hypophosphorige Säure H₃PO₂ und/oder DMSO als Modifikator verwendet werden.

Eine weitere, besonders bevorzugte Ausführungsform des erfindungsgemässen Verfahrens ist dadurch gekennzeichnet, dass als Katalysator Platin auf Kohlenstoff (Pt/C) in Kombination mit hypophosphoriger Säure H₃PO₂ und/oder DMSO eingesetzt wird.

Die Reaktionstemperatur beträgt zum Beispiel bevorzugt -20°C bis 100°C und besonders bevorzugt 0°C bis 60°C.

Der Wasserstoffdruck kann bis zu 100 bar und bevorzugt 1 bis 20 bar betragen.

Die Reaktion kann in Anwesenheit von anorganischen Basen wie zum Beispiel Natriumhydrogencarbonat, Natriumcarbonat, Natriumphosphat, Kaliumphosphat, Dinatriumhydrogenphosphat, Dikaliumhydrogenphosphat oder tertiären Stickstoffbasen wie zum Beispiel Triethylamin als Säurefänger von gebildeten Halogenwasserstoffen durchgeführt werden. Es können stöchiometrische Mengen, bezogen auf Halogenameisensäureester, oder auch ein Überschuss eingesetzt werden. Bevorzugt werden anorganische Basen eingesetzt. Besonders bevorzugt ist die Verwendung schwacher, anorganischer Basen wie Dinatrium- oder Dikaliumhydrogenphosphat oder Natriumhydrogencarbonat.

Eine ganz besonders bevorzugte Ausführungsform des erfindungsgemässen Verfahrens dient der Herstellung eines Zwischenproduktes der Formel A des Fungizids Pyraclostrobin 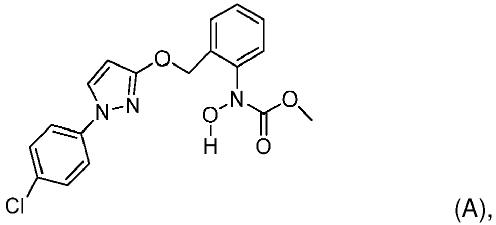 durch Hydrierung der entsprechenden Nitroverbindung der Formel B 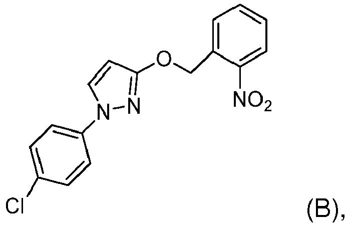 mit Wasserstoff in Gegenwart eines Hydrierkatalysators in einem inerten Lösungsmittel und in Gegenwart einer Base, das dadurch gekennzeichnet ist, dass es in Gegenwart mindestens stöchiometrischer Mengen eines Halogenameisensäureesters der Formel C₁-C₄-AlkylO-C(=O)-Hal durchgeführt wird.

Eine weitere, ganz besonders bevorzugte Ausführungsform des erfindungsgemässen Verfahrens dient der Herstellung von Methyl hydroxy(o-tolyl)carbamat durch Hydrierung von 2-Nitrotoluol mit Wasserstoff in Gegenwart eines Hydrierkatalysators in einem inerten Lösungsmittel und in Gegenwart einer Base, das dadurch gekennzeichnet ist, dass es in Gegenwart mindestens stöchiometrischer Mengen eines Halogenameisensäureesters der Formel C₁-C₄-AlkylO-C(=O)-Hal durchgeführt wird.

Das erfindungsgemässe Verfahren wird im Allgemeinen so durchgeführt, dass man Die Nitroverbindung und den Halogenameisensäureester mit dem Lösungsmittel vorlegt. Anschliessend werden der Katalysator und gegebenenfalls die Base zugegeben. Es ist aber ohne weiteres auch möglich, in umgekehrter Reihenfolge zu verfahren. Danach wird Wasserstoff aufgepresst und die Reaktion gestartet. Der Reaktionsverlauf kann durch die Wasserstoffaufnahme verfolgt werden. Wenn kein Wasserstoff mehr aufgenommen wird, ist das Verfahren beendet. Das Reaktionsgemisch wird dann in bekannter Weise aufgearbeitet wie zum Beispiel Filtration, Extraktion, Destillation oder Kristallisation. Das Verfahren kann in verschiedenen Reaktortypen kontinuierlich oder satzweise durchgeführt werden.

Die erfindungsgemäss herstellbaren N-substituierten Verbindungen sind entweder direkt aktive Substanzen oder Zwischenprodukte zur Herstellung solcher Substanzen, insbesondere im Bereich der Herstellung von Aromen und Geruchsstoffen, Pharmazeutika und Agrochemikalien und technischer Produkte wie Farbstoffe, Pigmente und Additive. Die direkte Herstellung des Handelsproduktes Pyraclostrobin ist schon zuvor erwähnt worden.

Die nachfolgenden Beispiele erläutern die Erfindung.

### Beispiel 1: Herstellung von Methyl hydroxy(phenyl)carbamat

In einem Glasgefäss legt man 50 mg 5% Pt-Kohle-Katalysator mit 15 mg 50%-iger wässriger hypophosphoriger Säure und 1 mL deionisiertem Wasser vor und rührt 10 Minuten.
In einer Parr^{®}-Glasflasche legt man 1,04 g Nitrobenzol in 30 mL Cyclopentylmethylether vor und gibt 1,68 g (1,4 Äquivalente) Dinatriumhydrogenphosphat sowie 1,04 g (1,3 Äquivalente) Chlorameisensäuremethylester dazu. Die Katalysatorsuspension spült man anschliessend mit 4 mL deionisiertem Wasser in die Parr^{®}-Glasflasche und hydriert 3 Stunden bei einer Temperatur von 22°C un d einem Wasserstoffdruck von 4 bar. Nach Inertisieren der Apparatur mit Stickstoff wird 1 mL Methanol dazugegeben und 15 Minuten gerührt um den überschüssigen Chlorameisensäuremethylester zu vernichten. Anschliessend werden die Feststoffe abfiltriert und mit 20 ml Cyclopentylmethylether gewaschen. Das Filtrat wird über Natriumsulfat getrocknet und im Vakuum bei 60°C eingedampft. Man erhält 1,43 g (101% der Theorie) Methyl hydroxy(phenyl)carbamat mit einer Reinheit von 91,7 % (Ausbeute 92,6%), welches noch 8,3% Methyl phenylcarbamat enthält (bestimmt mit HPLC (220 nm)).

### Beispiel 2: Herstellung von Methyl(2-(((1-(4-chlorophenyl)-1H-pyrazol-3-yl)oxy)methyl)phenyl)(hydroxy)carbamat

In einem Glasgefäss legt man 83 mg 5% Pt-Kohle-Katalysator mit 164 mg 50%-iger, wässriger hypophosphoriger Säure und 1 mL deionisiertem Wasser vor und rührt 10 Minuten.
In einer Parr^{®}-Glasflasche legt man 1,65 g 1-(4-Chlorophenyl)-3-((2-nitrobenzyl)oxy)-1H-pyrazol in 30 mL Cyclopentylmethylether vor und gibt 0,994g (1.4 Äquivalente) Dinatriumhydrogenphosphat sowie 0,614 g (1,3 Äquivalente) Chlorameisensäuremethylester dazu. Die Katalysatorsuspension spült man anschliessend mit 4 mL deionisiertem Wasser in die Parr^{®}-Glasflasche und hydriert 7 Stunden bei einer Temperatur 22°C und ei nem Wasserstoffdruck von 4 bar. Nach Inertisieren der Apparatur mit Stickstoff wird 1 mL Methanol dazugegeben und 15 Minuten gerührt um den überschüssigen Chlorameisensäuremethylester zu vernichten. Anschliessend werden die Feststoffe abfiltriert und mit 20 mL Cyclopentylmethylether gewaschen. Das Filtrat wird über Natriumsulfat getrocknet und im Vakuum bei < 1 mbar und 60°C eingedampft. Man erhält 1,80 g beigen Feststoff bestehend aus Methyl(2-(((1-(4-chlorophenyl)-1 H-pyrazol-3-yl)oxy)methyl)phenyl)(hydroxy)carbamat mit einer Reinheit nach HPLC (220nm) von 97%, welches nach HPLC und LC-MS noch 3% Methyl(2-(((1-(4-chlorophenyl)-1H-pyrazol-3-yl)oxy)methyl)phenyl)carbamat enthält. Daraus errechnet sich eine Ausbeute von 93% der Theorie.

### Beispiel 3: Herstellung von Methyl(2-(((1-(4-chlorophenyl)-1H-pyrazol-3-yl)oxy)methyl)phenyl)(hydroxy)carbamat

In einem Glasgefäss legt man 1086 mg 5% Pt-Kohle-Katalysator (Wassergehalt: 54%) mit 3 mL deionisiertem Wasser und 550 mg 50%-iger, wässriger hypophosphoriger Säure und vor und rührt 10 Minuten.
In einem 100 mL Rührautoklaven legt man 10.00 g 1-(4-Chlorophenyl)-3-((2-nitrobenzyl)oxy)-1H-pyrazol in 50 mL 2-Methyltetrahydrofuran vor und gibt 6.03 g (1.4 Äquivalente) Dinatriumhydrogenphosphat sowie 3.73 g (1,3 Äquivalente) Chlorameisensäuremethylester dazu. Die Katalysatorsuspension spült man anschliessend mit 2 mL deionisiertem Wasser in den Autoklaven und hydriert bei einer Temperatur 21°C und einem Wasserstoffdruck vo n 7 bar. Nach 1.5 Stunden kommt die Wasserstoffaufnahme zum Stillstand. Nach Inertisieren der Apparatur mit Stickstoff wird 1 mL Methanol dazugegeben und 20 Minuten gerührt um den überschüssigen Chlorameisensäuremethylester zu vernichten. Anschliessend wird das Reaktionsgemisch mit 20 mL 2-MeTHF und 5 mL Wasser aus dem Autoklaven gespült. Die wässrige Phase wird im Scheidetrichter abgetrennt. Die organische Phase wird mit 3 mL Wasser gewaschen und nach Abtrennung der wässrigen Phase über Natriumsulfat getrocknet, filtriert und am Rotationsverdampfer bei 60°C eingedampft. Der Rückstand wird eine Stunde bei 80°C / < 1 mbar getrocknet. Es werden 11.06g beiger Feststoff isoliert mit einer Reinheit nach HPLC (254nm) von 98.4%, entsprechend einer Ausbeute von 96%. Der Feststoff wird bei 60°C in 40 mL 2-Methyltetrah ydrofuran gelöst, daraus 30 mL abdestilliert und die erhaltene Lösung unter Rühren auf 15°C abgekühlt. Nach 30 Minuten rühren werden die erhaltenen Kristalle abgenutscht und mit 10 mL kaltem 2-MeTHF gewaschen. Es werden 9.46 g umkristallisiertes Methyl(2-(((1-(4-chlorophenyl)-1 H-pyrazol-3-yl)oxy)methyl) phenyl) (hydroxy) carbamat mit einer Reinheit nach HPLC (220nm und 254nm) von 100% erhalten. Die Mutterlauge wird eingedampft und der Rückstand eine Stunde bei 80°C / < 1 mbar getrocknet. Man erhält 1.54g rötlichen Feststoff, der gemäss HPLC (220 nm) 86.8% Methyl(2-(((1-(4-chlorophenyl)-1H-pyrazol-3-yl)oxy)methyl)phenyl)(hydroxy) carbamat und 9.8% Methyl(2-(((1-(4-chlorophenyl)-1H-pyrazol-3-yl)oxy)methyl)phenyl)carbamat enthält.

### Beispiel 4: Herstellung von Methyl hydroxy(o-tolyl)carbamat

In einem 22 mL Glasvial legt man 38 mg 5% Pt-Kohle-Katalysator (Wassergehalt: 54%) mit 0.7 mL deionisiertem Wasser und 19 mg 50%-iger, wässriger hypophosphoriger Säure vor und rührt 10 Minuten. Anschliessend werden 3.4 mL Tetrahydrofuran, 497 mg Dinatriumhydrogenphosphat und 307 mg Methylchloroformat dazu gegeben. Nach Spülen mit Argon und Wasserstoff wird das Gemisch während 7 Stunden unter 6 bar Wasserstoff bei 25°C gerührt. Die organische Phase wird anschliessend mittels HPLC und LC-MS analyisiert. Es werden 90% Methyl hydroxy(o-tolyl)carbamat und 7% Methyl (o-tolyl)carbamat nachgewiesen, sowie 3% eines unbekannten Nebenprodukts (Flächen-%, UV 220nm).

### Beispiele 5-11: Herstellung von Methyl hydroxy(o-tolyl)carbamat

Analog Beispiel 4 wurden verschiedene Lösungsmittel getestet.
Die nachfolgende Tabelle enthält die Resultate der HPLC-Analyse (Flächen-% bei 220 nm) der organischen Phase.

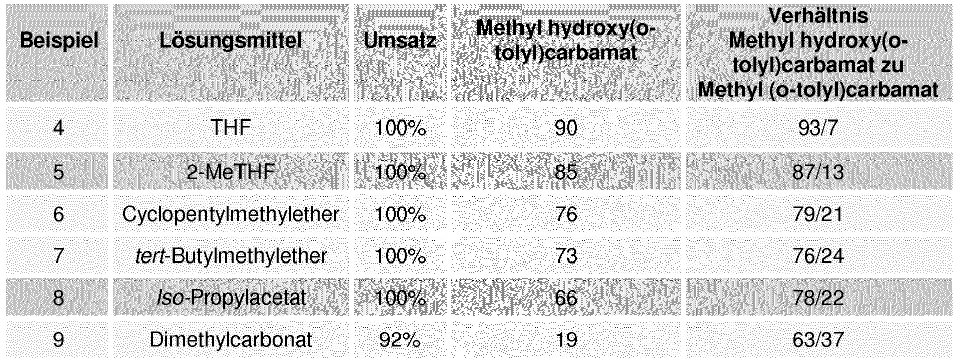

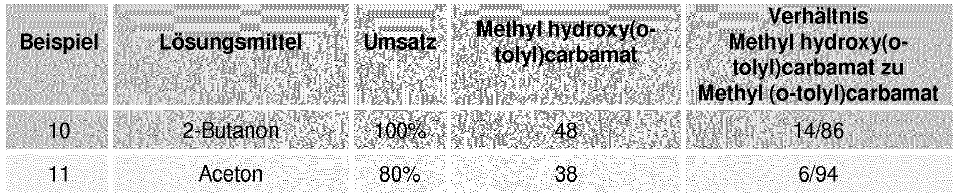

## Patentansprüche

1. Verfahren zur Herstellung von aromatischen Hydroxylaminen der Formel I
R-N(OH)-C(=O)-(O)R₁ (I),
worin R ein aromatischer Rest ist und R₁ einen Kohlenwasserstoffrest bedeutet, durch Hydrierung von aromatischen Nitroverbindungen der Formel II
R-NO₂ (II),
worin R die zuvor angegebene Bedeutung hat, mit einem Hydrierkatalystor und Wasserstoff in einem aprotischen Lösungsmittel, **dadurch gekennzeichnet, dass** man die Reaktion in Anwesenheit wenigstens stöchiometrischer Mengen einer Verbindung der Formel III
Y-C(=O)-(O)R₁ (III),
worin Y Halogen ist und R₁ die zuvor angegebene Bedeutung hat, durchführt.

2. Verfahren gemäss Anspruch 1, **dadurch gekennzeichnet, dass** es sich bei der Gruppe R um aromatische Kohlenwasserstoffe oder Heteroaromaten handelt, oder um kondensierte oder verknüpfte Ringsysteme, und die Gruppe R unsubstituiert oder substituiert ist.

3. Verfahren gemäss Anspruch 2, **dadurch gekennzeichnet, dass** es sich bei der Gruppe R um mono- oder polycyclische aromatische Kohlenwasserstoffe oder Heteroaromaten handeln, deren Ringsysteme kondensiert oder verknüpft sind, und die Gruppe R unsubstituiert oder substituiert ist.

4. Verfahren gemäss Anspruch 1, **dadurch gekennzeichnet, dass** R₁ für lineares oder verzweigtes C₁-C₈-Alkyl, C₃-C₈-Cycloalkyl, Benzyl oder Phenyl steht.

5. Verfahren gemäss Anspruch 4, **dadurch gekennzeichnet, dass** R₁ C₁-C₄-Alkyl bedeutet.

6. Verfahren gemäss Anspruch 1, **dadurch gekennzeichnet, dass** es sich bei den Verbindungen der Formel III um C₁-C₄-AlkylO-CO-Cl handelt.

7. Verfahren gemäss Anspruch 1, **dadurch gekennzeichnet, dass** es sich bei den Verbindungen der Formel III um Chlorameisensäuremethylester handelt.

8. Verfahren gemäss Anspruch 1, **dadurch gekennzeichnet, dass** es in Gegenwart eines inerten aprotischen und apolaren oder polaren Lösungsmittels durchgeführt wird.

9. Verfahren gemäss Anspruch 1, **dadurch gekennzeichnet, dass** dem Reaktionsgemisch Wasser zugesetzt wird.

10. Verfahren gemäss Anspruch 1, **dadurch gekennzeichnet, dass** es sich bei dem Katalysator um einen Platinkatalysator handelt.

11. Verfahren gemäss Anspruch 1, **dadurch gekennzeichnet, dass** die Edelmetallkatalysatoren in Mengen von 0.1 bis 10 Gew.% verwendet werden, bezogen auf das Substrat.

12. Verfahren gemäss Anspruch 1, **dadurch gekennzeichnet, dass** Modifikatoren zum Katalysator oder zum Reaktionsgemisch gegeben werden.

13. Verfahren gemäss Anspruch 12, **dadurch gekennzeichnet, dass** tertiäre Amine, Phosphorverbindungen oder Schwefelverbindungen oder Gemische davon als Modifikatoren zugegeben werden.

14. Verfahren gemäss Anspruch 13, **dadurch gekennzeichnet, dass** die Phosphorverbindungen ausgewählt sind aus der Gruppe Phosphine, Phosphinoxide und Phosphorsäuren.

15. Verfahren gemäss Anspruch 14, **dadurch gekennzeichnet, dass** die Phosphorverbindung hypophosphorige Säure H₃PO₂ ist.

16. Verfahren gemäss Anspruch 13, **dadurch gekennzeichnet, dass** die Schwefelverbindungen ausgewählt sind aus der Gruppe Sulfide, Thiole, Thioether, Sulfoxide, Thioharnstoffe und aromatische Schwefelverbindungen.

17. Verfahren gemäss Anspruch 14, **dadurch gekennzeichnet, dass** die Schwefelverbindung Dimethylsulfoxid ist.

18. Verfahren gemäss Anspruch 1, **dadurch gekennzeichnet, dass** als Katalysator Platin auf Kohlenstoff (Pt/C) in Kombination mit Dimethylsulfoxid und/oder hypophosphoriger Säure H₃PO₂ eingesetzt wird.

19. Verfahren gemäss Anspruch 15, **dadurch gekennzeichnet, dass** die hypophosphorige Säure oder deren Derivat im Verhältnis vom Modifikator zu Katalysator von 0,01 bis 20 zu 1 Gewichtsteilen, bevorzugt 0,1 bis 5 zu 1 Gewichtsteilen Katalysator verwendet wird.

20. Verfahren gemäss Anspruch 17, **dadurch gekennzeichnet, dass** Dimethylsulfoxid im Verhältnis von 0,1 bis 20 zu 1 Gewichtsteilen, bevorzugt 1 bis 6 zu 1 Gewichtsteilen Katalysator verwendet wird.

21. Verfahren gemäss Anspruch 1, **dadurch gekennzeichnet, dass** man als Lösungsmittel tert-Butylmethylether, tert-Butylethylether, tert-Amylmethylether, Cyclopentylmethylether, Tetrahydrofuran oder 2-Methyltetrahydrofuran verwendet.

22. Verfahren gemäss Anspruch 1, **dadurch gekennzeichnet, dass** der Wasserstoffdruck 1 bar bis 20 bar beträgt.

23. Verfahren gemäss Anspruch 1, **dadurch gekennzeichnet, dass** die Reaktionstemperatur 0°C bis 60°C beträgt.

24. Verfahren gemäss Anspruch 1, **dadurch gekennzeichnet, dass** zusätzlich eine Base zugegeben wird.

25. Verfahren gemäss Anspruch 24, **dadurch gekennzeichnet, dass** es sich bei der Base um Natriumhydrogencarbonat, Dinatriumhydrogenphosphat, Dikaliumhydrogenphosphat oder eine tertiäre Stickstoffbase handelt.

26. Verfahren gemäss Anspruch 1, **dadurch gekennzeichnet, dass** man eine Verbindung der Formel A 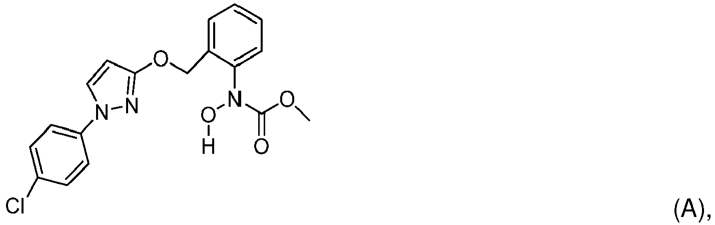 durch Katalytische Hydrierung der entsprechenden Nitroverbindung der Formel B 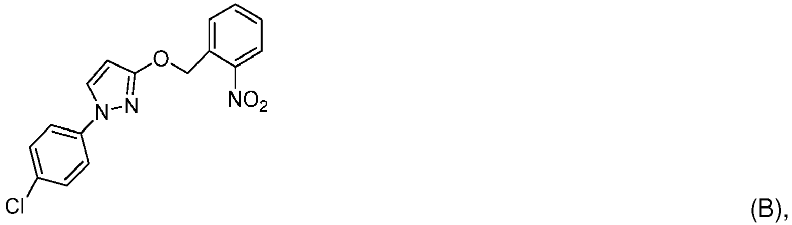 mit Wasserstoff in Gegenwart eines Edelmetall- oder Basismetallkatalysators in einem inerten Lösungsmittel und in Gegenwart einer anorganischen Base herstellt, **dadurch gekennzeichnet, dass** es in Gegenwart mindestens stöchiometrischer Mengen Chlorameisensäuremethylester der Formel MethylO-C(=O)-Cl durchgeführt wird.

27. Verfahren gemäss Anspruch 26, **dadurch gekennzeichnet, dass** der Katalysator ein mit hypophosphoriger Säure der Formel H₃PO₂ modifizierter Platin-Katalysator ist.

28. Verfahren gemäss Anspruch 26, **dadurch gekennzeichnet, dass** der Katalysator ein mit Dimethylsulfoxid und gegebenenfalls hypophosphoriger Säure der Formel H₃PO₂ modifizierter Platin-Katalysator ist.

29. Verfahren gemäss Anspruch 1, **dadurch gekennzeichnet, dass** man N-Methyl-hydroxy-(phenyl)carbamat durch Hydrierung von 2-Nitrotoluol mit Wasserstoff in Gegenwart eines Edelmetall- oder Basismetallkatalysators in einem inerten Lösungsmittel und in Gegenwart einer anorganischen Base herstellt, **dadurch gekennzeichnet, dass** es in Gegenwart mindestens stöchiometrischer Mengen Chlorameisensäuremethylester durchgeführt wird.

30. Verfahren gemäss Anspruch 29, **dadurch gekennzeichnet, dass** der Katalysator ein mit hypophosphoriger Säure der Formel H₃PO₂ modifizierter Platin-Katalysator ist.

31. Verfahren gemäss Anspruch 29, **dadurch gekennzeichnet, dass** der Katalysator ein mit Dimethylsulfoxid und gegebenenfalls hypophosphoriger Säure der Formel H₃PO₂ modifizierter Platin-Katalysator ist.
